# EUROPEAN PATENT APPLICATION

(11) **EP 4 834 231 A1**
(43) Date of publication of application: **04.11.2026**
(21) Application number: 25174022.1
(22) Date of filing: 02.05.2025
(51) Int. Cl.: A61B 6/03, A61B 6/50, A61B 6/00, G06T 7/00

(54) **PRODUCING A TISSUE DENSITY INDICATOR FOR A BREAST TISSUE OF A SUBJECT**

(71) Applicant: Koninklijke Philips N.V., 5656 AE Eindhoven (NL)
(72) Inventor: ERHARD, Klaus Alfred, Eindhoven (NL)
(74) Representative: Philips Intellectual Property & Standards

(57) **Abstract**

A tissue density indicator for a breast tissue of a subject in a medical procedure is produced by receiving three-dimensional (3D) medical imaging data resulting from scanning a chest of the subject using a 3D scanner. The 3D medical imaging data is processed to produce a 3D model of the breast tissue of the subject. The 3D model is then processed using a deforming procedure to produce a deformed 3D model, where the deforming procedure comprises processing the 3D model to simulate performing a mammography on the breast of the subject. The deformed 3D model is then processed to produce the tissue density indicator.

## Description

### FIELD OF THE INVENTION

The present disclosure relates to the field of medical imaging and, in particular, to medical imaging of breast tissue.

### BACKGROUND OF THE INVENTION

Breast tissue density is a significant risk factor for clinical analysis of breast tissue, as high breast tissue densities complicate detection of anomalies, e.g., nodules, nodes or cancers, through traditional 2D mammography due to overlapping tissue.

As such, there is an increasing use and reliance upon 3D imaging modalities, such as CT, MR and 3D ultrasound imaging, to image breast tissue. The 3D medical imaging data produced by such 3D imaging modalities may be processed to produce a volumetric measurement of breast tissue density. This volumetric measurement has been identified as being a more reliable quantitative breast density measurement, with higher inter-reader consistency, than breast tissue density measurements produced using traditional 2D mammography.

However, 3D imaging techniques generally suffer from the need to use expensive, bulky and time-consuming equipment to produce 3D medical imaging data. Such equipment may not be suitable for a wide variety of clinical scenarios and environments, e.g., breast screening at a clinic, health center or doctor's office.

### SUMMARY OF THE INVENTION

The invention is defined by the claims.

In accordance with a proposed approach, there is provided a computer-implemented method for producing a tissue density indicator for breast tissue of a subject in a medical procedure. The computer-implemented method comprises: receiving three-dimensional (3D) medical imaging data resulting from scanning a chest of the subject using a 3D scanner; processing the 3D medical imaging data to produce a 3D model of the breast tissue of the subject; processing the 3D model using a first deforming procedure to produce a first deformed 3D model, the first deforming procedure comprising processing the 3D model to simulate performing a mammography on the breast of the subject; and processing the first deformed 3D model to produce the tissue density indicator.

This method enables the generation of a tissue density indicator, comparable to what would be reported in a mammography examination, from 3D imaging data. The simulation of mammography on a 3D model allows for consistent and reproducible density assessments across different imaging modalities.

In particular, it has been recognized that comparing area-based measurements and/or indicators, e.g., produced using standard mammogram techniques with volumetric 3D measures or by processing 3D medical imaging such as from CT scans, introduces inconsistencies, because the 3D analysis will inherently provide lower density values. It has been recognized that there is currently an incomparability between a volumetric measure of breast density and an estimation derived from 2D imaging techniques, e.g., in a mammography. The proposed invention resolves this conflict by recomputing a breast density value from 3D medical imaging data, making it comparable to what would be reported in a mammography examination.

In some examples, the computer-implemented method further comprises projecting the first deformed model into a two-dimensional (2D) plane to produce first synthetic mammogram data; and processing the synthetic mammogram data to produce the tissue density indicator.

This approach allows for the creation of synthetic mammogram-like data from 3D imaging, thereby facilitating comparison with traditional mammography results. The synthetic data can be processed using existing or known mammogram analysis techniques, providing continuity with established clinical practices.

In some examples, projecting the first deformed model into the two-dimensional plane is performed using a ray-tracing technique. Ray-tracing provides an accurate simulation of X-ray propagation through breast tissue, thereby resulting in more accurate synthetic mammogram image data.

In some examples, the first deforming procedure comprises deforming the 3D model to simulate a first compression applied to the breast of the subject during a mammography. Simulating breast compression in the 3D model accounts for tissue displacement and density changes that occur during actual mammography. This results in more accurate tissue density indicators that better reflect or resemble the conditions under which clinical mammograms are obtained.

In some examples, the computer-implemented method further comprises processing the 3D model using a second deforming procedure to produce a second deformed 3D model, the second deforming procedure comprising processing the 3D model to simulate a second compression applied to the breast of the subject during the mammography, wherein the second compression is different from the first compression; and processing the first deformed 3D model and the second deformed 3D model to produce the tissue density indicator.

By simulating multiple compressions, this approach mimics the standard practice of obtaining multiple mammographic views. This multi-view simulation enhances the accuracy and robustness of the tissue density indicator, providing a more appropriate indicator of assessment of breast tissue density.

In some examples, the first compression and the second compression make an angle of approximately between 30° and 60° with respect to one another. This angular range between compressions closely approximates the standard views used in clinical mammography. It ensures that the simulated compressions capture different tissue orientations, thereby improving the accuracy of the tissue density indicator.

In some examples, the computer-implemented method further comprises: segmenting the 3D medical imaging data using a segmentation technique to produce a segmentation result; and processing the segmentation result to produce the 3D model.

Segmentation allows for precise delineation of breast tissue from surrounding structures in the 3D imaging data. This results in a more accurate 3D model focused specifically on breast tissue, improving the subsequent deformation and density analysis acts.

In some examples, the segmentation technique is configured to segment breast tissue in the 3D medical imaging data, such that the segmentation result comprises only breast tissue.

In some examples, the segmentation technique is configured to discriminate between an adipose breast tissue and a glandular breast tissue; and the computer-implemented method further comprises processing the adipose breast tissue and the glandular breast tissue to produce the 3D model.

In some examples, the computer-implemented method further comprises deforming the 3D model using at least one of: an affine transformation, a morphological operation, and a polynomial warping. These provide suitable and robust techniques for deforming a 3D model of a breast.

In some examples, the 3D medical imaging data comprises 3D CT imaging data. Utilizing CT imaging data leverages the high spatial resolution and tissue contrast provided by this modality. In particular, CT-based 3D models are able to capture detailed internal breast structures, improving the accuracy of the simulated mammography and resulting tissue density indicator.

In some examples, the tissue density indicator is a measure of breast tissue density, i.e., a density measure. Providing a quantitative measure of breast tissue density offers an objective and more granular assessment of breast composition. This provides useful information for making a clinical assessment of the subject.

In some examples, the computer-implemented method further comprises processing the 3D medical imaging data to produce a volumetric tissue density indicator.

A volumetric tissue density indicator provides a comprehensive 3D assessment of breast tissue composition. This volumetric approach can capture spatial variations in density that may be missed in 2D projections, potentially offering more nuanced insights into breast tissue characteristics.

There is also proposed a computer program product comprising computer program code means which, when executed on a device having processing circuitry, cause the processing circuitry to perform all of the acts of the method according to any of the preceding examples.

There is also proposed a device for producing a tissue density indicator for a breast tissue of a subject in a medical procedure. The device comprises: processing circuitry, and a memory containing instructions that, when executed by the processing circuitry, configure the processing circuitry to: receive three-dimensional (3D) medical imaging data resulting from scanning a chest of the subject using a 3D scanner; process the 3D medical imaging data to produce a 3D model of the breast tissue of the subject; process the 3D model using a deforming procedure to produce a deformed 3D model, the deforming procedure comprising processing the 3D model to simulate performing a mammography on the breast of the subject; and process the deformed 3D model to produce the tissue density indicator.

These and other aspects of the invention will be apparent from and elucidated with reference to the embodiment(s) described hereinafter.

### BRIEF DESCRIPTION OF THE DRAWINGS

For a better understanding of the invention and to show more clearly how it may be carried into effect, reference will now be made, by way of example only, to the accompanying drawings, in which:
Fig. 1 is a flowchart illustrating an inventive method according to one embodiment of the present invention; and
Fig. 2 illustrates a system comprising an inventive device according to one embodiment of the present invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for producing a tissue density indicator for breast tissue of a subject using 3D medical imaging data. The tissue density indicator resembles that produced during a mammogram. The method comprises processing the 3D medical imaging data to create a 3D model, deforming the 3D model to simulate mammography, and processing the deformed model to generate a tissue density indicator.

Various embodiments of the present invention provide approaches for producing a tissue density indicator for breast tissue of a subject. In this context, a tissue density indicator may comprise a piece of data indicative of the breast tissue density. The tissue density indicator may take the form of a piece of binary data, e.g., indicating whether or not the breast tissue is "dense" or "not dense", a piece of categorical data or a piece of numerical data, e.g., a measure of breast density.

One form of known and commonly used tissue density indicator is the categorical indicator defined in the Breast Imaging Reporting and Data System (BI-RADS ^{®}), which can take the following categories, commonly referred to as "a", "b", "c" or "d", respectively: a) The breasts are almost entirely fatty; b) There are scattered areas of fibroglandular density; c) The breasts are heterogeneously dense, which may obscure small masses; d) The breasts are extremely dense, which lowers the sensitivity of mammography. More details are given by ACR BI-RADS Atlas, Breast Imaging Reporting and Data System, 5th Edition, American College of Radiology, Reston, VA,

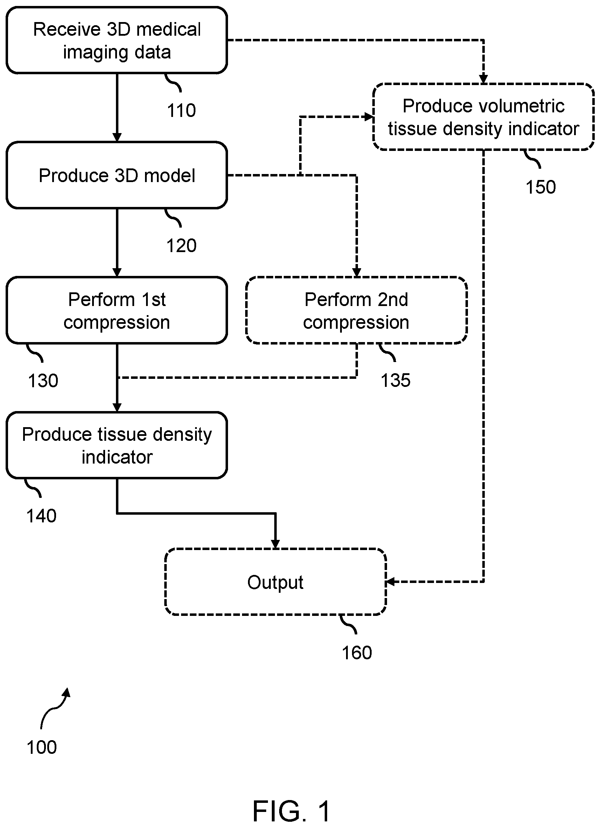

Fig. 1 is a flowchart illustrating an inventive computer-implemented method 100 for producing a tissue density indicator for breast tissue of a subject in accordance with one embodiment of the present invention. Each act of method 100 may be performed by processing circuitry of a device, examples of which are later described.

The computer-implemented method 100 comprises an act 110 of receiving 3D medical imaging data of the breast of the subject.

The 3D medical imaging data may be generated by any suitable 3D medical imaging system, e.g., according to any suitable 3D medical imaging modality. Examples include CT, MR, PET and 3D ultrasound imaging systems, although others will be readily apparent to the skilled person.

In the context of the present disclosure, 3D medical imaging data may comprise a 3D digital image, i.e., a 3D digital medical image. The 3D medical image data represents an imaged region of a subject, which may be a real-space region or a virtual-space region. For instance, the medical image data may comprise a plurality of discrete image elements, i.e., voxels, each representing a different subregion of the imaged region. Each discrete image element corresponds to a specific location in the imaged region and contains information about the intensity, color, material composition or any other property of said location.

The 3D medical imaging data may, for instance, comprise any form of 3D medical imaging data that can be produced by a 3D medical imaging system, such as ultrasound image data, CT image data, X-ray image data, PET image data, magnetic resonance imaging image data and so on.

Act 110 may comprise, for instance, receiving the 3D medical imaging data directly from the 3D medical imaging system. In other examples, act 110 may comprise receiving and/or retrieving the 3D medical imaging data from a data storage or memory configured to store the 3D medical imaging data.

The computer-implemented method 100 also comprises an act 120 of processing the 3D medical imaging data to produce a 3D model of the breast tissue of the subject.

In one example for performing act 120, the breast region of interest (ROI) in the 3D medical imaging data may be automatically identified using one or more model-based segmentation techniques, which are designed to accurately delineate the boundaries of the breast tissue from surrounding structures. The model-based segmentation produces a 3D model of the breast.

More particularly, act 120 may comprise segmenting the 3D medical imaging data using a segmentation technique to produce a segmentation result. The segmenting may comprise applying one or more image processing algorithms to isolate the breast tissue from other anatomical structures in the 3D medical imaging data to produce a segmentation result. Example image processing algorithms include edge detection, thresholding, watershed procedures, and morphological operations to define the breast boundaries. In some examples, machine learning techniques such as convolutional neural networks may be employed to improve the accuracy of the segmentation process.

In particular examples, the segmentation technique may be configured to segment breast tissue in the 3D medical imaging data, such that the segmentation result comprises only breast tissue. By focusing solely on breast tissue, the resulting 3D model can more accurately represent the internal structure and composition of the breast. This targeted segmentation approach helps eliminate potential confounding factors or artifacts that could arise from including non-breast tissues in any subsequent analysis.

The act 120 may comprise processing the segmentation result to produce the 3D model. This may be performed by applying a suitable 3D reconstruction technique to the segmented result. Example 3D reconstruction techniques include surface rendering, volume rendering, or mesh generation algorithms to create a 3D representation of the breast tissue structure.

In some examples, the segmentation technique is configured to discriminate between adipose breast tissue and glandular breast tissue. Accordingly, processing the segmentation result may comprise processing the segmented adipose breast tissue and the segmented glandular breast tissue to produce the 3D model.

By way of example, act 120 may comprise processing the 3D medical imaging data using a connected component analysis mechanism to identify breast components that are irrelevant for calculating breast density, such as skinfolds, pectoralis muscle, and implants, and excluding the identified breast components. Subsequently, an adaptive seeded watershed algorithm may be employed to discriminate glandular tissue from adipose tissue within the breast, thereby producing a 3D model of breast tissue.

As another example, the 3D medical imaging data may comprise multi-energy 3D medical imaging data, e.g., spectral 3D imaging data. It is recognized that different types of tissue will have different absorption or attenuation characteristics for different energy levels or types. This allows for the discrimination between adipose and glandular volume compartments through material decomposition utilizing spectral information from the different energy channels of the imaging data.

In general, a material decomposition process makes use of multi-energy 3D medical imaging data, i.e., imaging data having two or more channels containing imaging data captured at a respective two or more different energy levels. For example, in dual-energy CT imaging, two datasets are acquired at different kVp settings. These multi-energy 3D medical imaging data is then processed to separate the contributions of different materials based on their unique attenuation profiles.

For the purposes of act 120, this technique facilitates precise discrimination and identification of regions representing adipose and glandular tissues. In this way, the 3D model may incorporate information about different tissue types within the breast, such as adipose and glandular tissues, to provide a more comprehensive representation of the breast anatomy.

For instance, the 3D model comprises a plurality of 3D sub-models, each representing a different region of a particular type of breast tissue. For instance, each sub-model may represent a tissue region predicted to contain a single type of tissue, e.g., adipose or glandular tissue. This approach may allow for a more nuanced analysis of breast tissue composition and density distribution throughout the breast volume, particularly when processing the 3D model.

The positional relationship between the 3D sub-models is known or inherently defined, e.g., by the segmentation of the 3D medical imaging data.

In some examples, where the 3D medical imaging data is produced using a non-X-ray based imaging modality, such as an MR or 3D ultrasound imaging modality, the 3D model may be assigned with appropriate X-ray attenuation coefficients, such that the 3D model resembles one that would be produced if the 3D medical imaging data were produced using an X-ray based imaging modality, e.g., CT imaging.

By converting the 3D model to resemble X-ray based imaging, it allows for standardized processing and analysis across different imaging modalities, e.g., for ease of later comparison and assessment.

The process of assigning X-ray attenuation coefficients may comprise identifying different tissue types within the 3D model, e.g., adipose, glandular. This may be inherently performed if a plurality of 3D sub-models is produced. Subsequently, each tissue type may be assigned appropriate X-ray attenuation coefficients based on known values from literature or empirical measurements.

The computer-implemented method 100 also comprises an act 130 of processing the 3D model using a first deforming procedure to produce a first deformed 3D model, the first deforming procedure comprising processing the 3D model to simulate the performance of a mammography on the breast of the subject.

In some examples, the first deforming procedure may comprise deforming the 3D model to simulate a first compression applied to the breast of the subject during a mammography. In other words, the first deforming procedure may simulate the physical compression that occurs during a mammography examination. This simulation may involve applying virtual forces to the 3D model to mimic compression performed by the compression plates used in mammography.

The simulated first compression may be defined using a predefined force to be applied to a contact area of the breast. The magnitude of the predefined force used during a simulated first compression may be a predetermined value, e.g., a value between 100 N and 200N. Preferably, the magnitude of the simulated first compression is between 100 N and 140 N.

In some examples, the simulated first compression may be defined using a predefined pressure applied to the breast. The magnitude of the predefined pressure used during the simulated first compression may be a predetermined value, e.g., a value between 5 kPA and 20kPA, e.g., between 5 kPA and 15 kPA, e.g., 10 kPA.

In some examples, where the subject has previously undergone a mammogram, a recorded magnitude of compression during the previous mammogram is used to perform the simulated first compression.

In some examples, the first deforming procedure may comprise simulating the positioning of the breast during a mammography, which may include adjusting the orientation of the breast model to represent a particular mammographic view, e.g., the bilateral craniocaudal (CC) or mediolateral oblique (MLO) view.

More particularly, in some examples, the first deforming procedure may comprise using a finite-element simulation to deform the 3D model. The finite-element simulation may define elastic moduli for the 3D model or each 3D sub-model, e.g., as defined in literature, and simulate a typical mammographic compression force. Boundary conditions may be applied to the lower and upper compression paddles during the simulation.

Examples of suitable elastic moduli for breast tissue and different types of breast tissue are given in Krouskop, Thomas A., et al. "Elastic moduli of breast and prostate tissues under compression." Ultrasonic imaging 20.4 (1998): 260-274. However, other exemplary literature sources will be readily apparent to the appropriately skilled person.

In some examples, the transformation of the 3D model to produce the 3D model, specifically the simulation of the compression, may be performed using affine transformations, morphological operations, polynomial warping, or a combination of these methods. Approaches for modifying a 3D model to simulate a compression will be apparent to the skilled person, e.g., using an analogous approach to that disclosed in Liu, Yan-Lin, et al. "Simulation of breast compression in mammography using finite element analysis: A preliminary study." Radiation Physics and Chemistry 140 (2017): 295-299 or Li, Christina M., et al. "Three-dimensional computer generated breast phantom based on empirical data." Medical Imaging 2008: Physics of Medical Imaging. Vol. 6913. SPIE, 2008.

More specifically, one approach for deforming the 3D model may employ a finite-element modelling (FEM) technique as follows. First, the 3D model may be discretized into a mesh of smaller elements, e.g., (super-)voxels. One or more material properties, such as elasticity and compressibility, are assigned to each element, e.g., based on tissue type to distinguish between adipose and glandular tissues. Boundary conditions are defined to represent the compression plates and chest wall. Virtual forces are then applied to simulate the compression. The resulting system of equations is solved to determine the deformation of each element, producing a simulation of breast tissue compression during mammography.

Where the 3D model comprises a plurality of 3D sub-models, each representing tissue of a certain type, each sub-model may have different compression characteristics or elastic moduli to represent the different characteristics of the tissue represented by each sub-model. For instance, any 3D sub-models representing adipose tissue may be associated with a first elastic modulus, with any 3D sub-models representing glandular tissue being associated with a second elastic modulus, e.g., using the values defined in Krouskop 1998.

In such examples, if using the described finite-element modelling approach, each discretized element of the overall 3D model may be assigned its material property or properties based on into which 3D sub-model it falls.

The computer-implemented method also comprises an act 140 of processing at least the first deformed 3D model to produce the tissue density indicator. It will be clear that the tissue density indicator represents a 2D projected indicator of tissue density.

In one approach, act 140 is performed by projecting the first deformed model into a two-dimensional plane, e.g., using ray tracing or a similar technique, to produce first synthetic mammogram data and processing the first synthetic mammogram data to produce the tissue density indicator.

In some examples, processing the first synthetic mammogram data may be performed by using a watershed algorithm or by analyzing the known density distribution along each X-ray path, followed by a subsequent thresholding technique. This produces a measure of breast tissue density.

Processing the first synthetic mammogram to produce a measure of density may comprise using a thresholding algorithm, e.g. based on a Gaussian-Mixture Model, or k-means clustering, a region-growing algorithm, e.g. based on a water-shed algorithm, or machine-learning-based algorithms. These algorithms generate discrimination of the produced synthetic mammogram into areas of dense tissue and areas of predominantly fatty tissue, which may then be used to compute the fraction of dense tissue area over the complete projected breast region. This fraction of dense tissue area represents a measure of density.

Producing the measure of density may also include analyzing the known 3D density distribution to compute a projected 2D density map, e.g. by applying the thresholding operation along each projected X-ray path to generate a binary synthetic mammogram comprising either adipose or glandular pixels. The density measure can then be computed as the fraction of the dense tissue pixel over all pixels inside the projected breast.

In one example, act 140 is performed by processing the first synthetic mammogram data using a thresholding technique. In particular, the first synthetic mammogram data may be defined as comprising a plurality of tissue pixels, each taking a respective intensity value. A threshold value may be applied to classify each tissue pixel of the first synthetic mammogram data as either a dense tissue pixel or a non-dense tissue pixel.

The threshold value may be defined as a threshold value that discriminates between adipose (fatty) and glandular breast tissue. This threshold value may be determined through empirical studies or by using statistical methods that analyze the distribution of pixel intensities in a large dataset of mammograms.

As previously mentioned, a tissue density indicator may take a binary, categorical or numerical data format.

By way of example, the tissue density indicator may be produced as a binary value, such as "dense" or "not dense". This data format may be generated by applying a threshold to the density measure obtained from processing the synthetic mammogram data, e.g., using the previously disclosed technique. If the calculated density exceeds a predetermined threshold, the tissue may be classified as "dense", otherwise it may be classified as "not dense".

As another example, the tissue density indicator may be produced as a categorical value, such as the BI-RADS breast density categories (a, b, c, or d). This format may be generated by defining multiple thresholds or ranges for the density measure, with each range corresponding to a specific category. The calculated density measure may then be compared to these ranges to determine the appropriate category, e.g., by identifying into which range the calculated density measure forms.

Where the tissue density value takes a numeric value, the density measure, i.e., the measure of breast tissue density, may be directly used as the tissue density indicator.

It will be appreciated, in some examples, the tissue density indicator may be further processed to produce other indicators of tissue density. For instance, the tissue density indicator may take a numeric value, e.g., be the measure of breast tissue density, and the method 100 may further comprise processing the tissue density indicator to produce a further indicator of tissue density, e.g., in a binary or categorical data format, e.g., using any previously described approach.

Further optional acts and variations to previously described acts of the method 100 are hereafter described.

In some examples, the method 100 comprises processing (act 135) the 3D model using a second deforming procedure to produce a second deformed 3D model. The second deforming procedure comprises processing the 3D model to simulate a second compression applied to the breast of the subject during mammography. The second compression is different from the first compression.

The second deforming procedure is designed to mimic a different mammographic view or compression angle compared to the first deformation. This approach is analogous to standard mammography practices where multiple views, typically craniocaudal and mediolateral oblique, are taken to provide a more comprehensive assessment of breast tissue.

The first and second compressions may differ in compression angle, compression force, and/or compression direction.

In particular, the second compression might be applied at a different angle relative to the first, simulating different mammographic views. For instance, the first compression and the second compression may make an angle of approximately between 30° and 75° with respect to one another, e.g., approximately between 30° and 60° with respect to one another. More specifically, the second compression may make an angle of about 45° with respect to the first compression.

The act 140 of processing at least the first deformed 3D model may be modified to comprise processing at least the first deformed 3D model and the second deformed 3D model to produce the tissue density indicator.

In some examples, processing both deformed models may involve generating respective synthetic mammogram data for each deformed model, producing a density measure for each instance of synthetic mammogram data, e.g., using a previously disclosed approach, and then combining the density measures to produce a combined density measure. The combined density measure is a suitable example of a measure of breast tissue density for functioning as the tissue density indicator.

The combination of the density measures to produce the combined density measure may make use of any suitable combination technique, e.g., averaging, summing, weighted summing or other fusion algorithms. Typically, the average of the density measures functions as the combined density measure.

The combined density measure may function as the tissue density indicator. Alternatively, the combined density measure may be further processed to produce the tissue density indicator, e.g., to convert it into a binary or categorical data format, e.g., using the previously described approaches.

The method 100 may further comprise processing the tissue density indicator to produce a further indicator of tissue density, e.g., in a binary or categorical data format, using any previously described approach. This approach is particularly useful when the tissue density indicator is the measure of breast tissue density, i.e., takes a numeric value.

In some examples, the method 100 further comprises processing (act 150) the 3D medical imaging data to produce a volumetric tissue density indicator. This volumetric indicator provides a comprehensive measure of breast tissue density throughout the entire breast volume, offering complementary information to the 2D projected indicator.

The volumetric tissue density indicator can be calculated by processing the 3D medical imaging data or the 3D model to determine the relative proportions of dense (glandular and fibrous) tissue and non-dense (adipose) tissue within the entire breast volume. This may involve segmenting the 3D medical imaging data to isolate the breast tissue, then classifying each voxel as either dense or non-dense based on its intensity or attenuation values. In some examples, this is performed as a part of act 120, which information can be re-purposed for performing act 150.

One approach to calculating the volumetric tissue density indicator is to use the following formula: $VBD = \frac{{V}_{dense}}{{V}_{total}}$ (1) where VBD is the volumetric breast density, V_{dense} is the volume of dense tissue, and Vₜₒₜₐₗ is the total breast volume.

Other suitable examples for calculating a volumetric breast density from 3D medical imaging data or a 3D model will be readily apparent to the skilled person.

In some examples, the volumetric breast density may be categorized into discrete levels or grades, similar to the BI-RADS density categories used for 2D mammography, to produce the volumetric breast density indicator. These categories may be defined based on specific ranges of the calculated VBD value, providing a standardized way to classify breast density in volumetric terms.

In some examples, the volumetric breast density indicator may take a binary format, e.g., produced by processing the volumetric breast density (VBD). In particular, a thresholding approach may be used to reformat the volumetric breast density in a binary data format. In some cases, a predetermined threshold value may be set to distinguish between "dense" and "not dense" breast tissue. For example, if the calculated VBD exceeds this threshold, the volumetric tissue density indicator may be classified as "dense". Conversely, if the VBD falls below the threshold, it may be classified as "not dense".

The method 100 may further comprise an act 160 of outputting the tissue density indicator.

Outputting the tissue density indicator may involve displaying the tissue density indicator on a display device, storing it in a database, transmitting it to another system or device, or incorporating it into a medical report. In some cases, outputting may include presenting the tissue density indicator alongside other relevant patient information or imaging data to provide context for clinical decision-making.

In particular, act 160 may comprise controlling an output user interface to provide a visual representation of the tissue density indicator.

In some examples, act 160 further comprises outputting the volumetric tissue density indicator, if produced, alongside the tissue density indicator. This may provide a more comprehensive assessment of breast tissue density, combining both 2D information and 3D volumetric information. The combined output may enhance clinical decision-making by offering a more complete picture of breast tissue composition and density distribution.

It will be appreciated that the method 100 may be performed for each breast of the subject separately.

In some examples, the 3D medical imaging data received in act 110 may comprise 3D medical imaging data for both breasts of the subject. In such cases, the method may process the 3D medical imaging data to produce separate 3D models for each breast, e.g., for subsequent independent processing using acts 120, 130 and 140.

For instance, the act of processing the 3D medical imaging data to produce a 3D model may comprise generating two distinct 3D models - one for the left breast and one for the right breast. Each of these 3D models may then undergo the subsequent acts of the method independently.

Thus, the first deforming procedure may be applied to each 3D breast model separately, resulting in two first deformed 3D models. Similarly, if a second deforming procedure is employed, it may also be applied to each breast model independently.

When processing the deformed 3D models to produce tissue density indicators, this act may be performed for each breast separately. This approach may result in individual tissue density indicators for the left and right breasts.

In some examples, the method 100 may further comprise an act of comparing the tissue density indicators of the left and right breasts, not shown in the Figures. This comparison may provide additional clinical insights, as significant differences in density between the two breasts may be of interest to healthcare providers.

If performed, the act 160 may include presenting separate tissue density indicators for each breast, allowing for side-by-side comparison. This may involve displaying two sets of results on a display device, incorporating separate indicators for each breast in a medical report, or storing individual results for each breast in a database.

The skilled person would be readily capable of developing a device for carrying out any herein described method. Thus, each act of the flow chart may represent a different action performed by a processing system of a device, and may be performed by a respective module of the processing system of the device.

There is, therefore, proposed a device for producing a tissue density indicator for breast tissue of a breast of a subject in a medical procedure. The device comprises processing circuitry, and a memory containing instructions that, when executed by the processing circuitry, configure the processing circuitry to perform any herein proposed method.

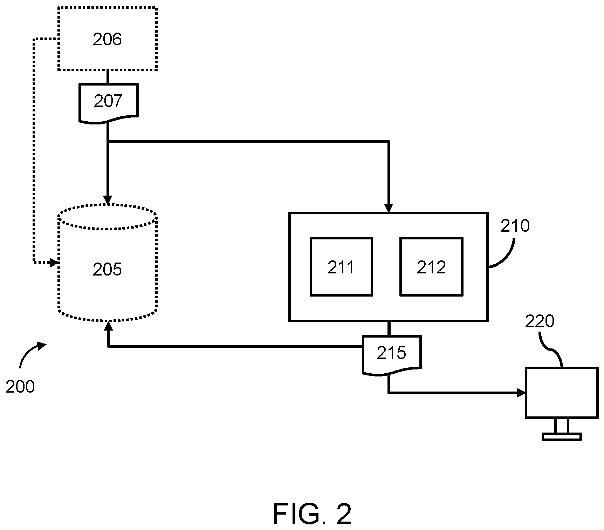

Fig. 2 illustrates a system 200 in which proposed device may be employed, for the purposes of improved contextual understanding.

The system 200 comprises a device 210 that includes processing circuitry 211 and a memory 212. The memory 212 contains instructions that, when executed by the processing circuitry, configure the processing circuitry to perform any herein disclosed method, thereby producing a tissue density indicator 215.

The system 200 may comprise a medical imaging system 206 configured to generate the 3D medical imaging data 207, which may be received or retrieved by the device 210.

The system 200 may comprise a memory unit 205 configured to store the 3D medical imaging data 207. The processing circuitry, in performing act 110, may retrieve the 3D medical imaging data 207 from the memory unit 205 or directly from the medical imaging system 206.

In some examples, the processing circuity, if performing act 160, is configured to store the tissue density indicator(s) in the memory unit 205.

In some examples, the system comprises an output user interface 220 configured to provide a visual representation of the tissue density indicator 215 and/or the 3D medical imaging data 207. In particular, the memory 212 may contain instructions that, when executed by the processing circuitry 211, configure the processing circuitry 211 to control the output user interface 220 to provide a visual representation of the tissue density indicator and/or the 3D medical imaging data.

The processing circuitry 211 can be implemented in numerous ways, with software and/or hardware, to perform the various functions required. A processor is one example of processing circuitry which employs one or more microprocessors that may be programmed using software, e.g., microcode, to perform the required functions. The processing circuitry may however be implemented with or without employing a processor, and also may be implemented as a combination of dedicated hardware to perform some functions and a processor, e.g., one or more programmed microprocessors and associated circuitry, to perform other functions.

Examples of processing circuitry components that may be employed in various embodiments of the present disclosure include, but are not limited to, conventional microprocessors, application specific integrated circuits (ASICs), and field-programmable gate arrays (FPGAs).

The memory 212 may comprise one or more storage media such as volatile and nonvolatile computer memory such as RAM, PROM, EPROM, and EEPROM. The memory is encoded with instructions, e.g., one or more programs that, when executed by the processing circuitry, perform the required functions. Various storage media may be fixed within the processing circuitry or may be transportable, such that the instructions, e.g., one or more programs, stored thereon can be loaded into processing circuitry.

It will be understood that disclosed methods are preferably computer-implemented methods. As such, there is also proposed the concept of a computer program comprising code means for implementing any described method when said program is run on a device having processing circuitry, such as a computer. Thus, different portions, lines or blocks of code of a computer program according to an embodiment may be executed by processing circuitry or computer to perform any herein described method.

There is also proposed a non-transitory storage medium that stores or carries a computer program or computer code which, when executed on a device having processing circuitry, cause the processing circuitry to perform any herein described method.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or acts, and the indefinite article "a" or "an" does not exclude a plurality.

Functions implemented by processing circuitry may be implemented by a single processor or by multiple separate processing units which may together be considered to constitute "processing circuitry". Such processing units may in some cases be remote from each other and communicate with each other in a wired or wireless manner.

The mere fact that certain indicators are recited in mutually different dependent claims does not indicate that a combination of these indicators cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A computer-implemented method for producing a tissue density indicator for a breast tissue of a subject in a medical procedure, the computer-implemented method comprising:
receiving three-dimensional (3D) medical imaging data resulting from scanning a chest of the subject using a 3D scanner;
processing the 3D medical imaging data to produce a 3D model of the breast tissue of the subject;
processing the 3D model using a first deforming procedure to produce a first deformed 3D model, the first deforming procedure comprising processing the 3D model to simulate performing a mammography on the breast of the subject; and
processing the first deformed 3D model to produce the tissue density indicator.

2. The computer-implemented method of claim 1, further comprising:
projecting the first deformed 3D model into a two-dimensional (2D) plane to produce synthetic mammogram data; and
processing the synthetic mammogram data to produce the tissue density indicator.

3. The computer-implemented method of claim 2, wherein projecting the first deformed 3D model into the 2D plane is performed using a ray-tracing technique.

4. The computer-implemented method of any one of claims 1 to 3, wherein the first deforming procedure comprises deforming the 3D model to simulate a first compression applied to the breast of the subject during the mammography.

5. The computer-implemented method of claim 4, further comprising:
processing the 3D model using a second deforming procedure to produce a second deformed 3D model, wherein the second deforming procedure comprises processing the 3D model to simulate a second compression applied to the breast of the subject during the mammography, wherein the second compression is different from the first compression; and processing the first deformed 3D model and the second deformed 3D model to produce the tissue density indicator.

6. The computer-implemented method of claim 5, wherein the first compression and the second compression make an angle of approximately between 30° and 60° with respect to one another.

7. The computer-implemented method of any one of claims 1 to 6, further comprising:
segmenting the 3D medical imaging data using a segmentation technique to produce a segmentation result; and
processing the segmentation result to produce the 3D model.

8. The computer-implemented method of claim 7, wherein the segmentation technique is configured to segment the breast tissue in the 3D medical imaging data, such that the segmentation result comprises only the breast tissue.

9. The computer-implemented method of any one of claims 7 or 8, wherein:
the segmentation technique is configured to discriminate between an adipose breast tissue and a glandular breast tissue; and
further comprising processing the adipose breast tissue and the glandular breast tissue to produce the 3D model.

10. The computer-implemented method of any one of claims 1 to 9, further comprising deforming the 3D model using at least one of: an affine transformation, a morphological operation, and a polynomial warping.

11. The computer-implemented method of any one of claims 1 to 10, wherein the 3D medical imaging data comprises 3D computed tomography (CT) imaging data.

12. The computer-implemented method of any one of claims 1 to 11, wherein the tissue density indicator is a measure of breast tissue density.

13. The computer-implemented method of any one of claims 1 to 12, further comprising processing the 3D medical imaging data to produce a volumetric tissue density indicator.

14. A computer program product comprising computer program code means which, when executed on a device having processing circuitry, cause the processing circuitry to perform the method according to any one of claims 1 to 13.

15. A device for producing a tissue density indicator for a breast tissue of a subject in a medical procedure, the device comprising:
processing circuitry; and
a memory containing instructions that, when executed by the processing circuitry, configure the processing circuitry to:
receive three-dimensional (3D) medical imaging data resulting from scanning a chest of the subject using a 3D scanner;
process the 3D medical imaging data to produce a 3D model of the breast tissue of the subject;
process the 3D model using a deforming procedure to produce a deformed 3D model, the deforming procedure comprising processing the 3D model to simulate performing a mammography on the breast of the subject; and
process the deformed 3D model to produce the tissue density indicator.
